# EUROPEAN PATENT SPECIFICATION

(11) **EP 2 242 507 B1**
(45) Date of publication and mention of the grant of the patent: **20.02.2013**
(21) Application number: 08762837.6
(22) Date of filing: 12.06.2008
(51) Int. Cl.: A61K 38/47, A61K 9/50, A61P 19/08

(54) **FORMULATION AND METHOD FOR THE PREVENTION AND TREATMENT OF SKELETAL MANIFESTATION OF GAUCHER'S DISEASE**
FORMULIERUNG UND VERFAHREN ZUR VORBEUGUNG UND BEHANDLUNG DER SKELETTMANIFESTATION VON MORBUS GAUCHER
FORMULATION ET PROCÉDÉ POUR LA PRÉVENTION ET LE TRAITEMENT DE MANIFESTATION SQUELETTIQUE DE LA MALADIE DE GAUCHER

(30) Priority: 13.02.2008 FR 0850910; 13.02.2008 US 28295
(43) Date of publication of application: 27.10.2010
(73) Proprietor: Erytech Pharma, 69008 Lyon (FR)
(72) Inventor: BOURGEAUX, Vanessa, F-69008 Lyon (FR); DUFOUR, Emmanuelle, F-69003 Lyon (FR); GODFRIN, Yann, F-69004 Lyon (FR)
(74) Representative: Colombet, Alain André
(86) International application number: PCT/IB2008/001504
(87) International publication number: WO 2009/101467

(56) References cited:
- FR-A- 2 873 925
- BOUVIER ET AL: "14. A novel approach for a specific delivery of glucocerebrosidase in bone marrow Gaucher cells" MOLECULAR GENETICS AND METABOLISM, ACADEMIC PRESS, SAN DIEGO, CA, US, vol. 93, no. 2, 14 January 2008 (2008-01-14), page 17, XP022420647 ISSN: 1096-7192
- PIERIGE ET AL: "Cell-based drug delivery" ADVANCED DRUG DELIVERY REVIEWS, AMSTERDAM, NL, vol. 60, no. 2, 13 December 2007 (2007-12-13), pages 286-295, XP022388014 ISSN: 0169-409X
- HAMIDI ET AL: "Applications of carrier erythrocytes in delivery of biopharmaceuticals" JOURNAL OF CONTROLLED RELEASE, ELSEVIER, AMSTERDAM, NL, vol. 118, no. 2, 2 March 2007 (2007-03-02), pages 145-160, XP005912142 ISSN: 0168-3659
- DALE G L ET AL: "Incorporation of glucocerebrosidase into Gaucher's disease monocytes in vitro." PROCEEDINGS OF THE NATIONAL ACADEMY OF SCIENCES OF THE UNITED STATES OF AMERICA JAN 1979, vol. 76, no. 1, January 1979 (1979-01), pages 473-475, XP009103029 ISSN: 0027-8424 cited in the application
- JAITELY V ET AL: "Resealed erythrocytes: Drug carrier potentials and biomedical applications" INDIAN DRUGS 1996 IN, vol. 33, no. 12, 1996, pages 589-594, XP009103058 ISSN: 0019-462X
- MILLÁN CARMEN GUTIÉRREZ ET AL: "Drug, enzyme and peptide delivery using erythrocytes as carriers." JOURNAL OF CONTROLLED RELEASE : OFFICIAL JOURNAL OF THE CONTROLLED RELEASE SOCIETY 20 FEB 2004, vol. 95, no. 1, 20 February 2004 (2004-02-20), pages 27-49, XP004487812 ISSN: 0168-3659

## Description

The invention relates to formulations and drugs for use in the prevention and treatment of skeletal manifestation of Gaucher's disease.

Gaucher's disease is an autosomal recessive genetic disease caused by a deficiency of a lysosomal enzyme, beta-glucocerebrosidase. It is the most common lysosomal storage disease with a prevalence of approximately 1/100,000 among the general population, but seems to be more common among the Ashkenazi Jewish population.

The disease is caused by mutations in the beta-glucocerebrosidase gene. More than 100 mutations of this gene have been described with different rates of occurrence which explains the large heterogeneity in the clinical presentation.

The beta-glucocerebrosidase hydrolyses glucosylceramide to form ceramide and glucose. The glucosylceramide is a complex glycolipid derived from the degradation of the cell membranes of red and white blood cells.

Due to the deficiency of beta-glucocerebrosidase, the undegraded glucosylceramide (or glucocerebroside) accumulates in the lysosome of macrophages of the reticuloendothelial system, principally in the liver, the spleen and bone marrow. Said macrophages loaded with glucocerebroside are called Gaucher cells. Said accumulation is the cause of the main clinical manifestations of the disease: hepatomegaly, splenomegaly and bone complications. If the bone is affected, this may be manifested by deformities, osteopenia or osteonecrosis; bone is affected in 80 % of patients.

Today, treatment of Gaucher's disease is based on two separate therapeutic strategies which aim to limit the storage of glucosylceramide so as to prevent the life-threatening effects linked to the accumulation thereof. The first of these strategies is substitutive enzymatic treatment with imiglucerase which is the reference treatment and is used in the first instance. The second, substrate reduction therapy with miglustat, is less effective and has more significant side effects. This therapy is thus reserved for an indication in the second instance when imiglucerase is no longer tolerated.

The aim of treatment by exogenous enzyme uptake (imiglucerase) is to fill the deficiency of glucocerebrosidase. Initially extracted, today the enzyme is recombinant and produced in CHO cells. It is also modified so as to expose mannose residues at its oligosaccharide chains and thus be recognised by the mannose receptors present on the surface of macrophages.

Treatment is begun at a dosage of 120 UI/kg/month by perfusions of 60 UI/kg every 15 days. Response to enzymatic therapy is generally good with an improvement in hepatosplenomegaly and haematological anomalies within a few months. The reduction in hepatic volume is, on average, between 20 and 30 % after 6 and 12 months of treatment respectively and the regression of splenic volume may reach 50 % after 12 months. The amount of haemoglobin increased to 1.5 g/dl within six months and normalised within a year, the number of platelets doubling within twelve months (Germain DP, Pathologie Biologie, 2004; 52: 343-350). Despite good general tolerance, between 10 and 15 % of patients develop antibodies to the enzyme and 50 % of these patients have pruritus or urticaria during perfusions of the enzyme.

Although enzyme replacement therapy makes it possible to improve haematological anomalies and hepatosplenomegaly within a few months, its efficacy is a lot more limited with regard to correcting bone manifestations of the disease. Bone pain gradually subsides after 24 months but objective improvement in skeletal manifestation, manifested by an increase in mineral bone density and a decrease in medullary infiltration, is only observed after 3 to 4 years of therapy (Poll LW et coll., The British Journal of Radiology, 2002; Suppl. 1: A25-A36). A study published by Wenstrup et Coll. (Wenstrup et coll., Journal of Bone and Mineral Research, 2007; 22: 119-126) synthesising the results obtained during an 8-year follow-up of 502 patients (160 not treated and 342 treated with the enzyme) showed that no improvement in mineral bone density was observed after 2 years of treatment A significant increase in bone density was observed after 4 and a half years. There is no return to a normal situation, however, until after 8 years of treatment.

Skeletal manifestation is among the most disabling features of Gaucher's disease. Patients suffer from bone pain and, in some cases, bone crises. The proportion of patients whose mobility is reduced is 20 %. Radiological examinations show bone Erlenmeyer flask deformity, osteopenia occasionally responsible for fractures, osteonecrosis and infiltration of the bone marrow (Wenstrup RJ et coll., The British Journal of Radiology, 2002; Suppl. I: A2-A12). The negative impact of these complications on patients' quality of life is significant. Consequently, there is a real need to develop new treatment methods which allow better management of skeletal manifestation of Gaucher's disease

Developments have been based on encapsulating glucocerebrosidase in erythrocytes with a view to improving the pharmacological properties of the enzyme.

Ihler et coll. (P.N.A.S. USA, 1973, 70, 9: 2663-2666) have shown that it is possible to encapsulate enzymes with a high molecular weight (beta-glucosidase and beta-galactosidase) in erythrocytes by means of a lysis-resealing process. The authors believe that beta-glucocerebrosidase could also be encapsulated in erythrocytes for the treatment of Gaucher's disease. Degradation of the membrane of said erythrocytes may also be envisaged with a view to targeting the macrophages of the spleen.

In Prog Clin Biol Res. 1982; 95: 655-667, Ihler reviews the studies regarding encapsulation of glucocerebrosidase in erythrocytes. The author recalls that free glucocerebrosidase has a half life of between 1 and 3 hours at 37°C in vitro and that, stabilised by the addition of DTT (dithiothreitol) or BSA (Bovine Serum Albumin), its half life increases to between 12 and 13 hours. Encapsulation in erythrocytes increases the half life of the enzyme in vitro to between 15 and 20 hours. The half life of the enzyme encapsulated in the erythrocytes increases to 400 hours in the presence of glucose. The author suggests that encapsulation of the enzyme may protect it until it is introduced into Gaucher cells and that this introduction could take place by erythrophagocytosis. He recalls that erythrophagocytosis may be enabled by different techniques, such as neuraminidase treatment, thermal treatment, binding of antibodies, and treatment by means of reagents reacting with sulfhydryl. The author also shows that the treatment of red blood cells with glutaraldehyde makes it possible to promote their elimination by macrophages of the spleen or of the liver, depending on the concentration of glutaraldehyde (table 5). This author writes at a time long before clinical application which is still very uncertain and does not address targeting of the bone marrow.

Another team clinically tested the use of a suspension of red blood cells encapsulating glucocerebrosidase (Beutler E et coll., P.N.A.S. USA, 1977, 74, 10: 4620-4623). One patient in the advanced stages of Gaucher's disease underwent 5 cycles of treatment alternating administration of free glucocerebrosidase and glucocerebrosidase encapsulated in erythrocytes over a period of 11 months. Clinical observation of the patient revealed a slight decrease in the size of the liver as well as a decrease in the levels of glucosylceramide in blood cells. The patient eventually died. A clinical benefit of the treatment has not been reported.

More recently, Bax et coll. carried out experiments aimed at optimising the parameters of encapsulation of glucocerebrosidase (Bax BE et coll. Biochem Soc Trans. 1996; 24(3): 441 S). The following year, the same team published the results of a pilot study aimed at testing the efficacy of beta-glucocerebrosidase encapsulated in erythrocytes in a patient suffering from Gaucher's disease and having undergone a splenectomy (Bain MD, Bax BE, Webster AD et Chalmers RA. Clin. Sci. (1997) 92, 3P). No clinical improvement in the patient's condition was recorded in this study.

Although glucocerebrosidase has been encapsulated in red blood cells and the theoretical benefit of the encapsulated enzyme as well as a targeting of the liver and of the spleen have been declared, the clinical efficacy even of this formulation remains to be seen. Furthermore, it is not possible to predict the capacity of a formulation of this type to prevent or improve the treatment of skeletal manifestation of Gaucher's disease. In addition, the possibility, on the one hand, of effectively targeting the bone marrow and, on the other hand, the development of means to target the bone marrow and to promote erythrophagocytosis by the macrophages and Gaucher cells possibly present in the bone marrow have not been described.

The objective of the invention is therefore to provide a solution which makes it possible to prevent and/or treat of skeletal manifestation of Gaucher's disease.

Another object of the invention is to provide a treatment of erythrocytes making it possible to target the macrophages and Gaucher cells in the bone marrow.

The invention thus relates to a formulation comprising glucocerebrosidase encapsulated in erythrocytes, wherein the erythrocytes encapsulating the glucocerebrosidase have been subjected to a chemical treatment with a bis(sulphosuccinimidyl) suberate (BS3) solution so as to promote the targeting of macrophages and/or Gaucher cells in the bone marrow for use in the prevention or treatment of skeletal manifestation of Gaucher's disease. In particular, the formulation makes it possible to introduce the enzyme into the lysosomal compartment of said cells. It comprises erythrocytes and glucocerebrosidase encapsulated inside said erythrocytes.

The invention also provides means for the prevention of skeletal manifestation of Gaucher's disease in patients who have not yet developed a manifestation of this type. Said patients are healthy but are likely to develop Gaucher's disease. Patients may also be suffering from Gaucher's disease with no actual or detectable skeletal manifestation.

The invention also provides means for treating of skeletal manifestation of Gaucher's disease in patients who have developed this manifestation, whatever its stage, early or advanced.

The invention also provides means for the prevention of skeletal manifestation of Gaucher's disease in patients who have not yet developed such a manifestation. Said patients are healthy but are likely to develop Gaucher's disease. Patients may also be suffering from Gaucher's disease with no actual or detectable skeletal manifestation.

The invention also provides means for the treatment of skeletal manifestation of Gaucher's disease in patients who have already developed this manifestation, whatever its stage, early or advanced.

The enzyme glucocerebrosidase (EC 3.2.1.45) is also called glucosylceramidase, β-glucosidase, or D-glucosyl-N-acylsphingosine glucohydrolase. It may be natural, synthetic, artificial or recombinant. The enzyme is currently recombinant and produced in CHO cells. Said recombinant origin is recommended. The notion of glucocerebrosidase also includes derivatives prepared so as to facilitate recognition by the macrophages, for example derivatives exposing mannose residues at their oligosaccharide chains. One enzyme which may be used is that which is marketed by Genzyme under the name Cerezyme®.

The formulation for use according to the invention is in the form of an erythrocyte suspension.

Preferably, the encapsulation of the enzyme is achieved by means of what is known as a lysis-resealing process.

In accordance with a characteristic of this process, a mixture of erythrocytes and glucocerebrosidase is produced. Said mixture is subjected to hypotonic conditions. The erythrocytes swell, opening the pores. The enzyme thus penetrates inside the erythrocytes. The isotonic conditions are then restored and the pores are thus resealed or reclosed in such a way that the erythrocytes encapsule the enzyme in a stable manner.

In accordance with the invention, the erythrocytes are treated with a chemical agent in conditions which promote the targeting of bone marrow cells (macrophages and/or Gaucher cells). Said targeting is to the disadvantage of macrophages and Gaucher cells in the liver.

The chemical treatment is carried out on the erythrocytes encapsulating the enzyme.

A chemical agent which is used according to the invention and compatible with human clinical use is bis(sulphosuccinimidyl) suberate (abbreviated to BS3 or BS³; CAS 82436-77-9). Advantageously, a solution of this agent is used.

In accordance with a characteristic of the process of BS3 treatment, the suspension of erythrocytes encapsulating the enzyme is contacted with the BS3 for an appropriate amount of time which could, in particular, be between approximately 10 min and approximately 1 hour. This time period is advantageously between approximately 15 min and approximately 45 min, preferably between approximately 20 and approximately 40 min, typically approximately 30 min.

In accordance with another characteristic of the process of BS3 treatment, this incubation is preferably carried out at ambient temperature, in particular between 18 and 25 °C.

In accordance with another characteristic of the process of BS3 treatment, the suspension of erythrocytes incorporating the enzyme is pre-washed with an appropriate buffer, for example PBS.

In accordance with another characteristic, the erythrocyte suspension is brought to a concentration between approximately 0.5x10⁶ and approximately 5x10⁶ cells/µl, typically between approximately 1x10⁶ and approximately 3x10⁶ cells/µl, before being contacted with the BS3 solution.

In accordance with another characteristic, a BS3 solution is used to obtain a final BS3 concentration between approximately 0.1 and approximately 6 mM, preferably between approximately 0.5 and approximately 3 mM, typically approximately 1 mM in the suspension. In particular, a BS3 solution of approximately 2 mM may be used. Preferably, a buffered BS3 solution is used, preferably containing glucose and a phosphate buffer in order to obtain the final desired BS3 concentration, in particular, of approximately 1 mM. In accordance with a characteristic, the pH of the buffered BS3 solution is advantageously between approximately 7.2 and approximately 7.6, preferably approximately 7.4. In accordance with another characteristic, the osmolarity of the buffered BS3 solution is between approximately 280 and approximately 320 mOsm.

The incubation may be stopped with an agent, such as Tris-HCl and the cells separated and possibly washed. For example, the mixture is centrifuged and the cells are washed. The cells are suspended again in an appropriate buffer, such as SAG-BSA. The mixture is left to incubate with said agent before separation, e.g. centrifugation, for a few minutes, in particular between 1 and 10 minutes, at ambient temperature.

The amount of enzyme encapsulated may, in particular, be between 1 and 50 UI of enzyme per ml of erythrocytes at a haematocrit of 100 %.

The suspension may be ready to use and have a haematocrit suitable for administration without dilution.

The suspension may also be conditioned in such a way that it must be diluted before administration.

According to the invention, the haematocrit of the suspension ready to use is advantageously between approximately 40 and approximately 70 %, preferably between approximately 45 and approximately 55 %, even more preferably approximately 50 %.

In its form to be diluted, the haematocrit may be greater, in particular between approximately 60 and approximately 90 %.

The suspension is preferably conditioned at a volume of approximately 10 to approximately 250 ml. The conditioning is preferably carried out in a blood bag (or pouch) of the type suitable for a blood transfusion. The amount of enzyme encapsulated corresponding to the medical prescription is preferably contained inside the blood bag.

The blood bag contains, for example, between 10 and 10,000 UI of enzyme, in particular between 200 and 5,000 UI.

In accordance with a characteristic of the invention, the erythrocytes to be administered are suspended in a pharmaceutically acceptable saline solution (for example standard environment for red blood cells, in particular solution containing NaCl and one or more ingredients selected from glucose, dextrose, adenine and mannitol; e.g. SAG-mannitol or ADsol). This solution ensures that the erythrocytes are conserved and may also include a conservation additive, such as L-carnitine.

The invention therefore provides means for introducing glucocerebrosidase into macrophages and/or Gaucher cells in the bone marrow. The formulation may, in particular, introduce the enzyme into the lysosomal compartment of said cells. These means comprise administration to the patient of a formulation or drug for use according to the invention.

Also disclosed herein is a method for preventing and/or treating skeletal manifestation of Gaucher's disease, in which an effective dose of a formulation or a drug for use according to the invention is administered to a patient in need thereof.

According to a first embodiment, the method described herein aims to prevent the skeletal manifestation of Gaucher's disease in patients who have not yet developed a manifestation of this type. Said patients are healthy but are likely to develop Gaucher's disease. Patients may also be suffering from Gaucher's disease with no actual or detectable skeletal manifestation.

According to a second embodiment, the method described herein aims to treat the skeletal manifestation of Gaucher's disease in patients who have developed this manifestation, whatever its stage, early or advanced.

In accordance with a particular embodiment, shock therapy is carried out on a patient. The object of shock therapy is to avoid the occurrence of skeletal manifestation or to slow its progression or even to stop and advantageously regress or eliminate said skeletal manifestation. This treatment may constitute a treatment in the first instance for a patient presenting to his doctor for the first time a risk for developing or signs of Gaucher's disease. The patient may also be resistant to conventional methods of treatment, for example a patient who has become resistant after a certain period of treatment by conventional methods. Said shock therapy may be recommended for long-term treatment with one shock administration or with a plurality of shock administrations at specific intervals.

In accordance with the invention, the formulation or drug is to be administered by intravenous or intra-arterial injection and preferably by perfusion from a blood bag or the like. Administration is typically carried out intravenously into the arm or by central catheter.

In particular, between approximately 10 and approximately 250 ml of formulation (one dose) according to the invention is administered. Perfusion is preferably used from 50 ml.

Treatment comprises administration of one dose or of a plurality of doses in accordance with the decided method. The method may allow for several monthly, bi-monthly, quarterly, biannual or annual administrations over the recommended course of treatment.

The techniques which make it possible to encapsulate active ingredients in red blood cells are known and the fundamental technique by means of lysis-resealing, which is preferred in this case, is described in patents EP-A-101 341 and EP-A-679 101, to which the person skilled in the art may refer. According to this technique, the primary compartment of a dialysis element (for example dialysis tube or dialysis cartridge) may be supplied with an erythrocyte suspension, whilst the secondary compartment contains an aqueous solution which is hypotonic relative to the erythrocyte suspension so as to lyse said erythrocytes; next, in a resealing unit, the resealing of the erythrocytes in the presence of the enzyme is induced by increasing the osmotic and/or oncotic pressure, then an erythrocyte suspension containing the enzyme is collected. In accordance with a characteristic of the invention, it is preferable to lyse an erythrocyte suspension already containing the glucocerebrosidase to be encapsulated.

The erythrocyte suspension encapsulating the glucocerebrosidase is particularly likely to be obtained by:
1 - suspending packed red blood cells in an isotonic solution with a haematocrit level equal to or greater than 65 %, then refrigerating between + 1 and + 8 °C,
2 - carrying out a method of lysis and internalisation of the glucocerebrosidase at a temperature constantly maintained between + 1 and + 8°C, comprising the passing of the erythrocyte suspension with a haematocrit level equal to or greater than 65 % and a hypotonic lysis solution refrigerated between + 1 and + 8°C into a dialysis tube or cartridge (a cartridge is preferred); the lysis parameters being adjusted as a function of the osmotic fragility measured previously; and
3 - carrying out a re-sealing process in the presence of a hypertonic solution, at an increased temperature, in particular between + 30 and + 42°C.

In a preferred variant, the method described in WO-A-2006/016247 which allows the enzyme to be encapsulated in a performant, reproducible, reliable and stable manner may be used. The suspension of erythrocytes encapsulating the glucocerebrosidase is thus likely to be obtained by;
1 - suspending packed red blood cells in an isotonic solution with a haematocrit level equal to or great than 65 %, then refrigerating between + 1 and + 8°C,
2 - measuring the osmotic fragility based on a sample of erythrocytes of said same packed red blood cells, it being possible to carry out steps 1 and 2 in any order (including simultaneously),
3 - carrying out a method of lysis and internalisation of the enzyme, in particular within the same chamber, at a temperature constantly maintained between + 1 and + 8 °C, comprising the passing of the erythrocyte suspension with a haematocrit level equal to or greater than 65 % and a hypotonic lysis solution refrigerated between + 1 and + 8°C into a dialysis tube or cartridge (a cartridge is preferred); the lysis parameters being adjusted as a function of the osmotic fragility measured previously; and
4 - carrying out a resealing method within a second chamber in the presence of a hypertonic solution at an increased temperature, in particular between + 30 and + 42°C.

### "Internalisation" means penetration of the enzyme inside erythrocytes.

In particular, for dialysis, packed red blood cells are suspended in an isotonic solution with an increased haematocrit level, equal to or greater than 65 %, and preferably equal to or greater than 70 %, and said suspension is refrigerated between + 1 and + 8 °C, preferably between + 2 and + 6 °C, typically at approximately 4°C. In accordance with a particular procedure, the haematocrit level is between 65 and 80 %, preferably between 70 and 80 %.

When it is measured, the osmotic fragility is advantageously measured on the erythrocytes just before the lysis step, in the presence of or in the absence of the enzyme in the suspension. The erythrocytes or the suspension containing them is/are advantageously at a temperature which is close to or identical to the temperature selected for lysis. According to another advantageous characteristic of the invention, the measurement of osmotic fragility obtained is quickly used, that is to say that the lysis process is carried out shortly after the sample has been taken. Preferably, the time period between obtaining the sample and beginning lysis is lower than or equal to 30 minutes, preferably even lower than or equal to 25 and even to 20 minutes.

With regard to the manner of carrying out the lysis-resealing process, with measurements and consideration of the osmotic fragility, the person skilled in the art may refer to WO-A-2006/016247 for more details.

The present invention will now be described in greater detail with reference to embodiments which are included by way of non-limiting examples.

### 1/ Coupling of beta-glucocerebrosidase to Oregon Green® 488

The coupling of the beta-glucocerebrosidase to Oregon Green® 488 was carried out according to publication Van Patten et al., 2007, Glycobiology, 17 : 467-478. However, an enzyme/fluorochrome ratio of between 1:50 and 1:20 was used instead of 1:10 as in the publication.

### 2/ Encapsulation of the beta-glucocerebrosidase by hypotonic column dialysis:

The beta-glucocerebrosidase (60 kDa), fluorescent (example 1) or not, was encapsulated in murine (mouse OF1) or human red blood cells (RBCs) by the method of hypotonic column dialysis. The blood was pre-centrifuged then washed three times in NaCl 0.9 %. Before starting dialysis the haematocrit was brought to 70 % in the presence of the enzyme which was added to a final concentration of 6 UI / ml. The RBCs were dialysed at a flow rate of 2 ml/min against a lysis buffer with low osmolarity (contra flow at 15 ml/min). The lysed RBCs exiting the column were resealed by adding a solution with high osmolarity and incubating for 30 minutes at 37°C. After resealing, the red blood cells were washed twice in NaCl 0.9 % glucose 0.2 %, then once with Sag-Mannitol supplemented with BSA (6 %) for the murine RBCs and only with Sag-Mannitol for the human RBCs. The red blood cells were brought to a haematocrit of 50 % to constitute the finished product.

### 3/ Final 1 mM BS3 treatment:

After resealing, the RBCs were washed twice in glucosed PBS, then diluted to 1.7x10⁶ cells/µl before being contacted with a 2 mM BS³ solution containing phosphate buffer 50 mM with a pH of 7.4, NaCl 56 mM and glucose 0.09 %. The RBCs were incubated for 30 minutes at ambient temperature then the reaction was stopped by adding a volume of Tris 20 mM, NaCl 150 mM. After 5 minutes of centrifugation, the RBCs were washed once with glucosed PBS, then once with Sag-Mannitol supplemented with BSA (6 %) for the murine RBCs and only with Sag-Mannitol for the human RBCs. The red blood cells were brought to a haematocrit of 50 % to constitute the finished product.

The red blood cells encapsulating the beta-glucocerebrosidase are called RBC-Gluco. The encapsulation produces RBC-Gluco at a concentration of approximately 2 UI of beta-glucocerebrosidase/ml of RBC at 100 % haematocrit.

During the encapsulation process, the total blood, the washed RBCs, the RBCs mixed with the beta-glucocerebrosidase (before dialysis) and the RBCs loaded with beta-glucocerebrosidase (after dialysis) were checked for:
- Haematocrit (Ht)
- Average globular volume (AGV)
- Average corpuscular concentration of haemoglobin (ACCH)
- Total haemoglobin concentration
- Cell count

Aliquots of cell suspensions were pre-washed before and after hypotonic dialysis to measure the enzyme activity of the beta-glucocerebrosidase.

The dosage of beta-glucocerebrosidase was derived from the publication Ron et al., 2005, "ER retention and degradation as the molecular basis underlying Gaucher disease heterogeneity", Hum. Mol. Genet., 14: 2387-2398.

The following results were obtained (Table 1):

| | human | | mouse | |
|---|---|---|---|---|
| | RBC-Gluco | BS3-treated RBC-Gluco | RBC-Gluco | BS3-treated RBC-Gluco |
| Stability of glucocerebrosidase activity in vitro after 24h at 4 °C | D0 : 1.25 Ul/ml | D0 : 1.67 UI/ml | D0 : 1.25 UI/ml | D0 : 1.21 UI/ml |
| | D1 : 1.27UI/ml | D1 : 1.67 UI/ml | D1 : 1.22 UI/ml | D1 : 1.18 UI/ml |
| Average corpuscular concentration of glucocerebrosidase (UI/ml RBC 100 % haematocrit) | 2.32 | 2.8 | 2.4 | 2.3 |
| Average globular content of glucocerebrosidase (UI/10⁹ RBC) | 0.26 | 0.27 | 0.13 | 0.12 |
| Yield of glucocerebrosidase encapsulation (%) | 39 | 46 | 40 | 39 |
| Cell yield (%) | 61 | 55 | 65 | 53 |

### 4/ Study of phagocytosis:

Fluorescent glucocerebrosidase (beta-glucocerebrosidase coupled to Oregon Green® 488) was encapsulated in mouse red blood cells by the method of column dialysis, and treated (BS3-treated RBC-Gluco) or not treated (RBC-Gluco) with BS3 1 mM. The mouse red blood cells OF1 loaded with glucocerebrosidase and treated or not treated with BS3 1 mM were injected intravenously into normal mice C57BI/6 (100x10⁹ RBC/kg of mouse). Normal mice were also injected with an amount of free fluorescent enzyme equivalent to that encapsulated. Control mice were injected with SAG Mannitol + BSA 6 %.

1 h 30 after injection, the mice were killed and the two femoral bones of the mice were removed. The cells of the bone marrow were recovered by injecting complete medium (RPMI 1640 medium, supplemented with Fetal Bovin Serum 10 %, Hepes 10 mM, Natrium Pyruvate 1 mM, β-mercaptoethanol 50 µm, Penicilline 100 U/mL, Streptomycine 100 µg/mL ; concentration are final concentrations) with a syringe. After lysis of the red blood cells, the bone marrow cells were counted and labelled with antibodies coupled to fluorochromes and specific to the macrophages (F4/80, CD11b) and to the dendritic cells (CD11c). The percentage of phagocyte cells (macrophages and dendritic cells) having phagocytosed the fluorescent enzyme encapsulated inside the red blood cells was measured by flow cytometry (FC500 Beckman Coulter).

The following results were obtained (Table 2):

| | phagocyte cells |
|---|---|
| Free glucocerebrosidase | 3.5% |
| RBC-Gluco | 3.8% |
| BS3-treated RBC-Gluco | 5.7% |

### 5/ Immunohistochemical double labelling:

The fluorochrome FITC-Dextran (70 kDa) was encapsulated in murine (mouse OFI) red blood cells by the method of hypotonic column dialysis. The blood was pre-centrifuged then washed three times with PBS. The haematocrit was brought to 70 % in the presence of FITC-Dextran and added to a final concentration of 8 mg/ml before starting dialysis. The RBCs were dialysed at a flow rate of 2 ml/min against a lysis buffer with low osmolarity (contra flow at 15 ml/min). The lysed RBCs exiting the column were resealed by adding a solution with high osmolarity and incubated for 30 minutes at 37°C. After two washes with glucosed PBS, the RBCs were diluted to 1.7x10⁶ cells/µl before being contacted with a BS³ solution 10 mM containing a phosphate buffer 50 mM with a pH of 7.4 and glucose 0.09 %. The RBCs were incubated for 30 minutes at ambient temperature then the reaction was stopped by adding a volume of Tris 20 mM, NaCl 140 mM. After being centrifuged for 5 minutes, the RBCs were washed once with glucosed PBS, then once with Sag-Mannitol supplemented with BSA (6 %). The red blood cells were brought to a haematocrit of 50 % to constitute the finished product which was injected on D1 into the mouse. The mouse was killed 1 h 30 after injection then the bone marrow was isolated from the femoral bones and placed inside a Tissue-Tek for nitrogen freezing. The cryostat cuts of 10 µm were carried out for immunohistochemical analysis. After fixing in acetone a double labelling was carried out showing the FITC (brown DAB) and the macrophages F4/80 (red, new fuschin).

Microscopic observation of the cuts shows colocalisation of macrophages and dextran. Observation confirms the incorporation of dextran by the macrophages by phagocytosis of red blood cells.

Analysis by flow cytometry (FC500 Beckman Coulter) gives the following information.

**Table 3: percentage of fluorescent cells in the bone marrow 1 h 30 after intravenous injection of RBCs into mice.**

| Treatments | Number of total fluorescent cells |
|---|---|
| RBC-Dextran. | 5.3% |
| BS3 RBC-Dextran | 7.4% |

The analysis by flow cytometry shows that 1 h 30 after injection 7 and 5 % of bone marrow cells were fluorescent in the case of BS3 treatment and in the case of no treatment respectively.

**Table 4 shows the percentage of phagocyte cells which phagocyted treated or untreated red blood cells.**

| Treatments | F4/80 macrophages | Dendritic cells |
|---|---|---|
| RBC-Dextran | 5.2% | 7.9% |
| BS3 RBC-Dextran | 9.5% | 16.3% |

Trapping and phagocytosis of the RBCs in the bone marrow is induced more quickly and is more significant with BS3 treatment than without treatment.

## Claims

1. Formulation comprising glucocerebrosidase encapsulated in erythrocytes, wherein the erythrocytes encapsulating the glucocerebrosidase have been subjected to a chemical treatment with a bis(sulphosuccinimidyl) suberate (BS3) solution so as to promote the targeting of macrophages and/or Gaucher cells in the bone marrow for use in the prevention or treatment of skeletal manifestation of Gaucher's disease.

2. Formulation for use according to claim 1, wherein the suspension of erythrocytes encapsulating the glucocerebrosidase is contacted with BS3 for between 10 min and 1 hour, in particular for between 15 main and 45 min, preferably for between 20 and 40 min, and typically for approximately 30 min.

3. Formulation for use according to either claim 1 or claim 2, wherein the incubation with BS3 is at ambient temperature.

4. Formulation for use according to any one of claims 1 to 34, wherein before incubation with BS3, the suspension of erythrocytes incorporating the glucocerebrosidase is washed with a suitable buffer, preferably with PBS.

5. Formulation for use according to any one of claims 1 to 4, wherein the suspension of erythrocytes encapsulating the glucocerebrosidase is brought to a concentration of between 0.5x10⁶ and 5 10⁶ cells/µl, preferably between 1x10⁶ and 3x10⁶ cells/µl, before being contacted with the BS3 solution.

6. Formulation for use according to any one of claims 1 to 5, wherein a BS3 solution is used to obtain a final BS3 concentration of between 0.1 and 6 mM, preferably between 0.5 and 3 mM, even more preferably of approximately 1 mM in the suspension.

7. Formulation for use according to any one claims 1 to 6, wherein a buffered BS3 solution having an osmolarity between 280 and 320 mOsm and a pH between 7.2 and 7.6, preferably 7.4, is used.

8. Formulation for use according to claim 7, wherein the BS3 solution comprises glucose and phosphate buffer.

9. Formulation for use according to any one of claims 1 to 8, wherein the amount of enzyme encapsulated is between 1 and 50 UI of enzyme per ml of erythrocytes at a haematocrit of 100 %.

10. Formulation for use according to any one of claims 1 to 9, wherein the suspension is conditioned in a blood bag containing between 10 and 10,000 UI glucocerebrosidase, in particular between 200 and 5,000 UI.

11. Formulation for use according to any one of claims 1 to 10, for use for the prevention of skeletal manifestation of Gaucher's disease in patients who have not yet developed a manifestation of this type.

12. Formulation for use according to claim 11, for use for healthy patients who are, however, likely to develop Gaucher's disease.

13. Formulation for use according to claim 11, for use for patients suffering from Gaucher's disease with no actual or detectable skeletal manifestation.

14. Formulation for use according to any one of claims 1 to 10, for the treatment of skeletal manifestation of Gaucher's disease in patients having developed said manifestation.

## Patentansprüche

1. Formulierung, die in Erythrozyten verkapselte Glukocerebrosidase enthält, zur Verwendung in der Vorbeugung oder Behandlung einer skeletalen Manifestation der Gaucher Erkrankung, wobei die Glukocerebrosidase-verkapselnden Erythrozyten einer chemischen Behandlung mit einer Lösung von Bis(sulfosuccinimidyl)-suberat (BS3) unterworfen wurden, um die Zielfindung von Makrophagen und/oder Gaucher-Zellen im Knochenmark zu fördern.

2. Formulierung zur Verwerdung gemäß Anspruch 1, wobei die Suspension an Glukocerebrosidase-verkapsetnden Erythrocyten zwischen 10 Min. und 1 Stunde, besonders zwischen 15 Min. und 45 Min., bevorzugt zwischen 20 und 40 Min., und typischerweise ungefähr 30 Min., mit BS3 kontaktiert wird.

3. Formulierung zur Verwerdung gemäß entweder Anspruch 1 oder 2, wobei die Inkubation mit BS3 bei Raumtemperatur stattfindet.

4. Formulierung zur Verwerdung gemäß einem der Ansprüche 1 bis 3, wobei vor der Inkubation mit BS3 die Suspension der Glukocerebrosidase-enthaltenden Erythrozyten mit einem geeigneten Puffer, bevorzugt mit PBS, gewaschen wird.

5. Formulierung zur Verwerdung gemäß einem der Ansprüche 1 bis 4, wobei die Suspension der Glukocerebrosidase-verkapselnden Erythrocyten vor der Kontaktierung mit der BS3-Lösung auf eine Konzentration zwischen 0,5·10⁶ und 5·10⁶ Zellen/µl, bevorzugt zwischen 1·10⁶ und 3·10⁶ Zellen/µl, eingestellt wird.

6. Formulierung zur Verwerdung gemäß einem der Anspruche 1 bis 5, wobei eine BS3-Lösung verwender wird, um eine endgültige BS3-Konzentration zwischen 0,1 und 6 mM, bevorzugt zwischen 0,5 und 3 mM, insbesondere ungefähr 1 mM, in der Suspension zu erhalten.

7. Formulierung zur Verwindung gemäß einem der Ansprüche 1 bis 6, wobei eine gepufferte BS3-Lösung mit einer Osmolarität zwischen 280 und 320 mOsm und einem pH zwischen 7,2 und 7,6, bevorzugt 7,4, verwendet wird.

8. Formulierung zur Verwerdung gemäß Anspruch 7, wobei die BS3-Lösung Glukose und einen Phosphatpuffer enthält.

9. Formulierung zur Verwendung gemäß einem der Ansprüche 1 bis 8, wobei die Menge an verkapseltem Enzym zwischen 1 und 50 UI Enzym pro ml Erythrozyten, bei einen Hämatokrit von 100%, beträgt.

10. Formierung zur Verwindung gemäß einen der Ansprüche 1 bis 9, wobei die Suspension in einen Blutbeutel konditioniert wird, welcher zwischen 10 und 10,000 UI, bevorzugt zwischen 200 bis 5,000 UI, Glukocerebrosidase enthält.

11. Formulierung zur Verwindung gemäß einem der Ansprüche 1 bis 10 zur Verwindung bei der Vorbeugung einer skeletalen Manifestation der Gaucher Erkrankung in Patienten, die noch keine Manifestation dieses Typs entwickelt haben.

12. Formulierung zur Verwendung gemäß Anspruch 11 zur Verwendung bei gesunden Patienten, die aber wahrscheinlich die Gaucher Erkrankung entwickeln könnten.

13. Formulierung zur Verwendung gemäß Anspruch 11 zur Verwendung bei Patienten, die an der Gaucher Erkrankung ohne gegenwärtiger oder detektierbarer skeletaler Manifestation leiden.

14. Formulierung zur Verwendung gemäß einem der Ansprüche 1 bis 10 zur Behandlung einer skeletalen Manifestation der Gaucher Erkrankung in Patienten, die besagte Manifestation entwickelt haben.

## Revendications

1. Formulation comprenant de la glucocérébrosidase encapsulée dans des érythrocytes dans laquelle les érythrocytes encapsulant la glucocérébrosidase ont été soumis à un traitement chimique avec une solution de bis(sulphosuccinimidyl) suberate (BS3) pour promouvoir le ciblage des macrophages et/ou des cellules de Gaucher dans la moelle osseuse, pour utilisation dans la prévention ou le traitement de l'atteinte osseuse de la maladie de Gaucher.

2. Formulation pour utilisation selon la revendication 1, dans laquelle la suspension d'érythrocytes encapsulant la glucocérébrosidase est mise en contact avec le BS3 pendant une durée comprise entre 10 min et 1 heure, notamment entre 15 min et 45 min, de préférence entre 20 et 40 min, typiquement de l'ordre de 30 min.

3. Formulation pour utilisation selon la revendication 1 ou 2, dans laquelle l'incubation avec le BS3 est à température ambiante.

4. Formulation pour utilisation selon l'une des revendications 1 à 3, dans laquelle, avant incubation avec le BS3, la suspension d'érythrocytes renfermant la glucocérébrosidase est lavée à l'aide d'un tampon approprié, de préférence du PBS.

5. Formulation pour utilisation selon l'une des revendications 1 à 4, dans laquelle la suspension d'érythrocytes encapsulant la glucocérébrosidase est amenée à une concentration comprise 0,5.10⁶ et 5.10⁶ cellules/µl, de préférence entre 1.10⁶ et 3.10⁶ cellules/µl, avant d'être mise en contact avec la solution de BS3.

6. Formulation pour utilisation selon l'une des revendications 1 à 5, dans laquelle on emploie une solution de BS3 pour obvenir une concentration finale en BS3 comprise entre 0,1 et 6 mM, de préférence entre 0,5 et 3 mM, mieux encore d'environ 1 mM dans la suspension.

7. Formulation pour utilisation selon l'une des revendications 1 à 6, dans laquelle on emploie une solution tamponnés de BS3 ayant une osmolarité comprise entre 280 et 320 mOsm et un pH compris entre 7,2 et 7,6, de préférence de 7,4.

8. Formulation pour utilisation selon la revendication 7, dans laquelle la solution de BS3 comprend du glucose et du tampon phosphate.

9. Formulation pour utilisation selon l'une des revendications 1 à 8, dans laquelle la quantité d'enzyme encapsulée est comprise entre 1 et 50 UI d'enzyme par ml d'érythrocytes à hématocrite 100%.

10. Formulation pour utilisation selon l'une des revendications 1 à 9, dans laquelle la suspension est conditionnés en poche de sang comprenant de 10 à 10 000 UI de glucocérébrosidase, notamment de 200 à 5 000 UI.

11. Formulation pour utilisation selon l'une quelconque des revendications 1 à 10, pour utilisation pour la prévention de l'atteinte osseuse de la maladie de Gaucher chez des patients n'ayant pas encore développé une telle atteinte.

12. Formulation pour utilisation selon la revendication 11, pour utilisation pour des patients sains mais susceptibles de développer la maladie de Gaucher.

13. Formulation pour utilisation selon la revendication 11, pour utilisation pour des patients atteints de la maladie de Gaucher, sans atteinte osseuse réelle ou détectable.

14. Formulation pour utilisation selon l'une quelconque des revendications 1 à 10, pour le traitement de l'atteinte osseuse de la maladie de Gaucher chez des patients ayant développé cette atteinte.
